(19) Europäisches Patentamt
European Patent Office
Office européen des brevets

(11) Veröffentlichungsnummer: **0 082 400 B1**

(12) # EUROPÄISCHE PATENTSCHRIFT

(45) Veröffentlichungstag der Patentschrift: **06.03.91**

(51) Int. Cl.[5]: **A01N 43/64**, A01N 43/50, //C07D249/08,C07D233/60

(21) Anmeldenummer: **82111293.5**

(22) Anmeldetag: **06.12.82**

(54) **Fungizide alpha-Azolylglykole.**

(30) Priorität: **18.12.81 DE 3150204**

(43) Veröffentlichungstag der Anmeldung:
**29.06.83 Patentblatt 83/26**

(45) Bekanntmachung des Hinweises auf die Patenterteilung:
**06.03.91 Patentblatt 91/10**

(84) Benannte Vertragsstaaten:
**AT BE CH DE FR GB IT LI NL SE**

(56) Entgegenhaltungen:

| | |
|---|---|
| EP-A- 0 021 345 | EP-A- 0 056 087 |
| DE-A- 2 105 490 | DE-A- 2 201 063 |
| DE-A- 2 324 424 | DE-A- 2 325 156 |
| DE-A- 2 333 355 | DE-A- 2 350 123 |

(73) Patentinhaber: **BASF Aktiengesellschaft**
**Carl-Bosch-Strasse 38**
**W-6700 Ludwigshafen(DE)**

(72) Erfinder: **Zeeh, Bernd, Dr.**
**Thorwaldsenstrasse 5**
**W-6700 Ludwigshafen(DE)**
Erfinder: **Ammermann, Eberhard, Dr.**
**Sachsenstrasse 3**
**W-6700 Ludwigshafen(DE)**
Erfinder: **Buschmann, Ernst, Dr.**
**Georg-Ludwig-Krebs-Strasse 10**
**W-6700 Ludwigshafen(DE)**
Erfinder: **Pommer, Ernst-Heinrich, Dr.**
**Berliner Platz 7**
**W-6703 Limburgerhof(DE)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

**Beschreibung**

Die vorliegende Erfindung betrifft die Verwendung fungizides Mittel, die alpha-Azolylglykole enthalten zur Bekämpfung von Fungi.

Es ist bekannt, O-Alkyl-N-1,2,4-triazolyl-acetale als Fungizide zu verwenden (DE-OS 24 31 407). Die fungizide Wirkung und Verträglichkeit gegenüber Kulturpflanzen wird jedoch nicht allen Anforderungen der Praxis gerecht. Es ist ferner bekannt, alpha-Azolylglykolderivate als Fungizide zu verwenden (EP-A-2l 345). In EP-A-56 087 werden alpha-Azolylglykole als Wachstumsregulatoren beschrieben.

Es wurde nun gefunden, daß alpha-Azolylglykole der Formel I

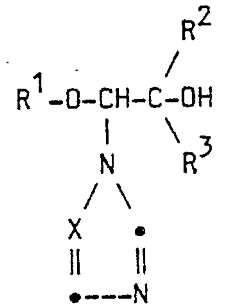

(I),

in welcher

$R^1$ $C_1$ - $C_6$ Alkyl,
$R^2$ C1 - C6 Alkyl oder gegebenfalls durch Halogen substituiertes Phenyl oder Biphenyl,
$R^3$ Wasserstoff, $C_1$ - $C_6$ Alkyl, $C_2$ - $C_6$ Alkenyl, $C_2$ - $C_6$ Alkinyl oder gegebenenfalls durch Halogen substituiertes Benzyl und
X CH oder H bedeutet

sowie deren Salze und Metallkomplexe gute fungizide Wirkung haben die der Wirkung der bekannten Triazolylderivate überlegen ist.

In der Formel I steht $R^1$ für einen verzweigten oder unverzweigten Alkylrest mit 1 bis 6 C-Atomen, wie Methyl, Ethyl, n-Propyl, iso-Propyl, n-Butyl, iso-Butyl, tert.-Butyl> n-Pentyl oder n-Hexyl.

$R^2$ steht für einen Alkylrest mit bis zu 6 Kohlenstoffatomen, der unverzweigt - wie Methyl, Ethyl, Propyl, Butyl oder Pentyl - oder verzweigt wie Isopropyl, tert.--Butyl oder 3-Methyl-butyl - sein kann. Ferner steht $R^2$ für einen unsubstituierten oder durch Halogen, vorzugsweise Chlor oder Brom, mono- oder disubstituierten Phenyl- oder Biphenylrest.

$R^3$ steht für Wasserstoff, für einen unverzweigten Alkylrest mit 1 bis 6 Kohlenstoffatomen - wie Methyl, Ethyl, Propyl, Butyl oder Pentyl -, für einen verzweigten Alkylrest mit 3 bis 6 Kohlenstoffatomen - wie Isopropyl oder iso-Butyl -, für einen Alkenyl- oder Alkinylrest mit 2 bis 6 Kohlenstoffatomen - wie Vinyl, Ethinyl, Propen-2-yl-1, Propin-2-yl-1 oder 3-Methyl-buten--2-yl -, oder für einen gegebenenfalls durch Halogen - wie beispielsweise Fluor oder Chlor - mono- oder disubstituierten Benzylrest.

Die alpha-Azolylglykole besitzen im Acetal-C-atom und - falls $R^2$ und $R^3$ verschieden sind - im Carbinol-C-atom je ein Asymmetriezentrum. Je nach Art des Substituenten $R^1$ kommen eventuell weitere Asymmetriezentren hinzu. Mit üblichen Trennmethoden können die Verbindungen in Form einheitlicher Enantiomerer bzw. Diastereomerer erhalten werden. In der Praxis kann man sowohl die einheitlichen Enantiomeren bzw. Diastereomeren wie auch die bei der Synthese üblicherweise anfallenden Gemische verwenden. Letztere werden bevorzugt verwendet. Die Erfindung umfaßt sowohl die einheitlichen Enantiomeren oder Diastereomeren als auch ihre Gemische.

Die alpha-Azolylglykole der Formel I lassen sich herstellen, indem man

a) ein Keton der Formel II

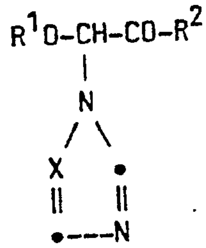

(II),

in der $R^1$, $R^2$ und X die oben erläuterten Bedeutungen haben, katalytisch oder mit komplexen Hydriden in Gegenwart eines Lösungsmittels und gegebenenfalls in Gegenwart eines Reaktionsbeschleunigers bei Temperaturen zwischen 0 und 100 °C reduziert oder

b) ein Keton der Formel II mit Grignardreagenzien der Formel IV

$$R^3MgHal \qquad IV,$$

in der $R^3$ die oben erläuterte Bedeutung hat und Hal für Chlor, Brom oder Iod steht, in Gegenwart inerter Lösungsmittel bei Temperaturen zwischen 0 und 80 °C umsetzt oder

c) ein Acetal der Formel

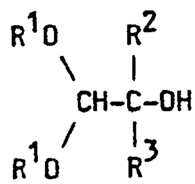

(V),

in der $R^1$, $R^2$ und $R^3$ die oben angegebenen Bedeutungen haben, mit anorganischen oder organischen Säurechloriden und anschließend mit einem Azol (Triazol, Imidazol) gegebenenfalls in Gegenwart eines Lösungsmittels und einer Base bei Temperaturen zwischen 0 und 100 °C umsetzt.

Die Ausgangsverbindungen II sind herstellbar durch Umsetzung von alpha-Halogenethern der Formel III

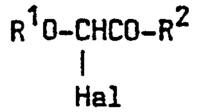

(III),

in der $R^1$ und $R^2$ die oben erwahnte Bedeutung haben und Hal Chlor oder Brom bedeutet, mit Azolen (1,2,4-Triazol, Imidazol) oder deren Alkali- oder Erdalkalisalzen gegebenenfalls in Gegenwart eines Lösungsmittels und einer Base bei Temperaturen zwischen 0 und 100 °C.

Die alpha-Halogenether der Formel III können nach bekannten Methoden hergestellt werden (vgl. DE-05 22 01 063 und B. Mylo, Chem. Ber. 44 (1911), S. 3212, Straus und Weber Ann. 498 (1932), S. 124). Ferner können die Verbindungen der Formel III auch durch Halogenierung (z.B. mit N-Brom-succinimid) der alpha-Alkoxyketone hergestellt werden.

Die Alkohole der Formel V sind teilweise bekannt wie z.B. das 1,1-Dimethoxy-2-methylbutin-3-ol-2 (DE-PS 17 68 877), oder das 1,1-Dimethoxy-2-methylbuten-3-ol-2 (DE-PS 11 15 238). Sie können aber auch nach bekannten Methoden hergestellt werden, indem man Ketone der Formel VI

$$(R^1O)_2CH-CO-R^2 \qquad VI$$

katalytisch oder mit komplexen Hydriden hydriert oder mit Grignardreagenzien der Formel IV umsetzt.

Geeignete anorganische oder organische Säurehalogenide für das Verfahren c) sind beispielsweise

Thionylchlorid, Acetylchlorid oder Acetylbromid. Darüber hinaus sind alle üblichen gut zugänglichen Säurehalogenide verwendbar.

Geeignete anorganische oder organische Basen, die gegebenenfalls auch als säurebindende Mittel in die Verfahren a) oder c) eingesetzt werden können, sind beispielsweise Alkali- und Erdalkalihydroxide - wie Natriumhydroxid, Kaliumhydroxid, Calciumhydroxid -, Alkalicarbonate - wie Kalium- oder Natriumcarbonat -, Alkalihydride - wie Natriumhydrid -, Alkali- oder Erdalkalialkoholate - wie Natriummethylat, Magnesiummethylat oder Natriumisopropylat - oder tertiäre Amine - wie Trimethylamin, Triethylamin, N,N-Dimethylanilin, N,N-Dimethylcyclohexylamin, N-Methylpiperidin oder Pyridin.

Ferner können die Azole (1,2,4-Triazol, Imidazol) selbst als Basen verwendet werden. Mit geeigneten Basen, zum Beispiel Alkalihydriden - wie Natriumhydrid -, Lithiumalkylen - wie Butyllithium -, oder Alkali- oder Erdalkalialkoholaten - wie Natriummethylat - können die Azole auch in einer vorgeschalteten Umsetzung zunächst in ihre Salze überführt und dann als solche zur Reaktion gebracht werden.

Zu den bevorzugten Lösungs- bzw. Verdünnungsmitteln gehören Halogenkohlenwasserstoffe - wie Methylenchlorid, Chloroform, 1,2-Dichlorethan, Chlorbenzol -, aliphatische oder aromatische Kohlenwasserstoffe - wie Cyclohexan, Petrolether, Benzol, Toluol, oder Xylole -, Ester - wie Essigsäureethylester -, Amide - wie Dimethylformamid -, Nitrile - wie Acetonitril -, Sulfoxide - wie Dimethylsulfoxid -, Ketone - wie Aceton oder Methylethylketon -, Ether - wie Diethylether, Tetrahydrofuran oder Dioxan -, oder entsprechende Gemische.

Als Reaktionsbeschleuniger kommen vorzugsweise Metallhalogenide - wie Kaliumiodid -, Kronenether, quartäre Ammoniumverbindungen - wie Tetrabutylammoniumiodid - oder Säuren oder Kombinationen dieser Reaktionsbeschleuniger in Frage.

Die Umsetzungen werden im allgemeinen bei Temperaturen zwischen 0 und 150° C in einem Zeitraum von 1 bis 60 Stunden durchgeführt, drucklos oder unter Druck, kontinuierlich oder diskontinuierlich.

Zur Isolierung der Verbindungen wird nach üblichen Methoden vorgegangen. Im allgemeinen bedürfen die anfallenden Produkte keiner weiteren Reinigung, sie können aber auch nach bekannten Methoden wie Umkristallisation, Extraktion, Destillation oder Chromatographie weiter gereinigt werden.

Falls gewünscht, werden die alpha-Azolylglykole der Formel I anschließend nach bekannten Verfahren in ihre für Pflanzen verträglichen Salze oder in ihre Metallkomplexe überführt.

Zur Salzbildung eignen sich beispielsweise Salzsäure, Bromwasserstoffsäure, Schwefelsäure, Salpetersäure, Phosphorsäure, Essigsäure oder Dodecylbenzolsulfonsäure. Die Wirksamkeit der Salze geht auf das Kation zurück, so daß die Wahl des Anions beliebig ist.

Metallkomplexe bilden sich durch Anlagerung der neuen Verbindungen an die Kationen von Metallsalzen. Besonders eignen sich hierfür Kupfer(II)chlorid, Kupfer(II)-sulfat, Kupfer(II)-nitrat, Zink(II)-chlorid, Eisen-(III)-chlorid, Mangan(II)-chlorid, Nickel(II)-bromid.

Die folgenden Beispiele schildern die Herstellung der neuen Substanzen:

Beispiel 1

a) Herstellung des Ausgangsmaterials:

Zu 48 g 1,1-Dimethoxy-3,3-dimethylbutan-2-on (vgl. J.B. Wright J. Am. Chem. Soc. 77 (1955), S. 4883) wurden unter Rühren 36,8 g Acetylbromid getropft. Dabei stieg die Temperatur auf 53° C an. Nach einstündigem Rühren wurde diese Lösung zu einer Lösung von 41,4 g Triazol in 100 ml Dimethylformamid und 100 ml Tetrahydrofuran getropft. Es wurde drei Stunden gerührt, dann das Reaktionsgemisch eingeengt, in Methylenchlorid aufgenommen und dreimal mit je 50 ml Wasser gewaschen. Die organische Phase wurde abgetrennt, getrocknet und eingeengt. Das verbleibende öl wurde über eine Kolonne destilliert. Zwischen 84 und 86° C/0,1 mbar gingen 44 g 1-(1',2',4'-Triazol-1'-yl)-1--methoxy-3,3-dimethyl-butan-2-on über.

b) Herstellung des Endproduktes:

39,4 g 1-(1',2',4'-Triazol-1'yl)-1-methoxy-3,3-dimethyl-butan-2-on wurden in 80 ml Methanol bei 10 bis 20° C portionsweise mit 4,5 g Natriumborhydrid versetzt. Anschließend wurde 1 Stunde bei Rückflußtemperatur gerührt. Das Reaktionsgemisch wurde in 80 ml Wasser eingerührt und dreimal mit je 100 ml Methylenchlorid extrahiert. Die organische Phase wurde abgetrennt, getrocknet und eingeengt. Das verbleibende öl kristallisierte aus Petrolether aus. Man erhielt so 34 g 1-(1',2',4'-Triazol-1'-yl)-1--methoxy-3,3-dimethyl-butanol-2 vom Schmelzpunkt 62 bis 64° C.

Beispiel 2

Zu einer Lösung von 0,2 Mol 4-Chlorphenylmagnesiumbromid (hergestellt aus 4,9 g Magnesium und 38,3 g 4-Bromchlor-benzol) in 150 ml Ether wurde eine Lösung von 1-(1',2',4'--Triazol-1'-yl)-1-methoxyaceton in 100 ml Ether getropft. Anschließend wurde 5 Stunden bei Rückflußtemperatur gerührt. Zu dem abgekühlten Reaktionsgemisch wurde 50 g Eis und dann 25 %ige wäßrige Ammoniumchloridlösung zugetropft, bis sich die Phasen klar trennten. Die organische Phase wurde abgetrennt und die wäßrige Phase zweimal mit je 100 ml Ether extrahiert. Die vereinigten Etherphasen wurden mit Wasser neutral gewaschen, getrocknet und eingeengt. Aus Petrolether erhielt man 23 g kristallines 1-(1',2',4'Triazol-1'-yl)-1-methoxy-2-(4'-chlorphenyl)-propanol-2 vom Schmelzpunkt 83 bis 82°C.

Beispiel 3

Zu 14,8 g 1,1-(Dimethoxy)-2-methyl-butanol-2 wurden unter Rühren 12,3 g Acetylbromid getropft. Dabei stieg die Temperatur bis 60°C an. Nach einer Stunde Rühren wurde dieses Reaktionsgemisch zu einer Lösung von 13,8 g Triazol in 100 ml Tetrahydrofuran und 50 ml Dimethylformamid getropft. Nach dem Rühren über Nacht wurde das Reaktionsgemisch eingeengt, in Methylenchlorid aufgenommen und dreimal mit je 50 ml Wasser gewaschen. Die organische Phase wurde getrocknet und eingeengt. Das verbleibende öl wurde destilliert. Bei 95 bis 110°C/0,4 mbar gingen 6 g 1-(1',2',4-Triazol-1'yl)-1-methoxy-2-methylbutanol-2 über.

Entsprechend wurden diejenigen in der folgenden Tabelle angegebenen Verbindungen der Formel I hergestellt, deren Schmelzpunkte (Fp) oder Siedepunkte (Kp) angegeben sind. Ihre Struktur wurde durch Infrarot- und KernresonanzSpektroskopie sowie durch Elementaranalyse gesichert. Die Verbindungen, bei denen keine Angaben zur physikalischen Chemie gemacht sind, können auf die gleiche Art und Weise wie bei den tatsächlich hergestellten Verbindungen erhalten werden; es kann erwartet werden, daß sie aufgrund ihrer ähnlichen Konstitution ähnliche Wirkungen wie die näher untersuchten Verbindungen haben.

| r Nr. | $R^1$ | $R^2$ | $R^3$ | X | Physikal. Konst. |
|---|---|---|---|---|---|
| 4 | $CH_3$ | $2,4-Cl_2-C_6H_3$ | H | N | Fp. 136-138°C |
| 5 | $C_2H_5$ | $2,4-Cl_2-C_6H_3$ | H | N | Fp. 113-115°C |
| 6 | $CH_3$ | $C_6H_5-$ | $CH_3$ | N | Fp. 102-104°C |
| 7 | $n-C_4H_9$ | $4-Cl-C_6H_4-$ | $CH_3$ | N | Fp. 58- 70°C |
| 8 | $iso-C_4H_9$ | $4-Cl-C_6H_4$ | $CH_3$ | N | Fp. 87- 90°C |
| 9 | $CH_3$ | $4-(C_6H_5)-C_6H_4$ | $CH_3$ | N | Fp. 176-178°C |
| 10 | $CH_3$ | $4-Cl-C_6H_4-CH_2$ | $CH_3$ | N | Fp. 110-112°C |
| 11 | $CH_3$ | $2,4-Cl_2-C_6H_3$ | $CH_3$ | N | Fp. 135-137°C |
| 12 | $C_2H_5$ | $2,4-Cl_2-C_6H_3$ | $CH_3$ | N | Fp. 150-152°C |
| 13 | $CH_3$ | $CH_3$ | H | N | Fp. 140-142°C |
| 14 | $CH_3$ | $CH_3$ | H | CH | |
| 15 | $CH_3$ | $tert.-C_4H_9$ | $CH_3$ | N | Fp. 85- 86°C |
| 16 | $CH_3$ | $tert.-C_4H_9$ | $CH=CH_2$ | N | Fp. 85°C |
| 17 | $CH_3$ | $tert.-C_4H_9$ | $CH_2-C_6H_5$ | N | Fp. 90- 92°C |
| 18 | $CH_3$ | $tert.-C_4H_9$ | $4-Cl-C_6H_4-CH_2-$ | N | Fp. 94- 96°C |
| 19 | $n-C_4H_9$ | $tert.-C_4H_9$ | $CH_3$ | N | $Kp_{0,01}$ 116-120°C |
| 20 | $n-C_4H_9$ | $tert.-C_4H_9$ | H | N | $Kp_{0,2}$ 100-125°C |
| 21 | $CH_3$ | $2,4-Cl_2C_6H_3$ | H | CH | Fp. 130-138°C |
| 22 | $C_2H_5$ | $2,4-Cl_2-C_6H_3$ | H | CH | Fp. 112°C |
| 23 | $CH_3$ | $C_6H_5$ | $CH_3$ | CH | |

| Nr. | $R^1$ | $R^2$ | $R^3$ | X | Physikal. Konst. |
|---|---|---|---|---|---|
| 24 | $n-C_4H_9$ | $4-Cl-C_6H_4$ | $CH_3$ | CH | |
| 25 | $CH_3$ | $2,4-Cl_2-C_6H_3$ | $CH=CH_2$ | CH | |
| 26 | $CH_3$ | $4-(C_6H_5)-C_6H_4$ | $CH_3$ | CH | Öl |
| 27 | $CH_3$ | $4-Cl-C_6H_4$ | $CH_3$ | CH | Fp. 134-136°C |
| 28 | $C_2H_5$ | $2,4-Cl_2-C_6H_3$ | $4-Cl-C_6H_4-CH_2$ | CH | |
| 29 | $CH_3$ | tert.-$C_4H_9$ | $CH_3$ | CH | |
| 30 | $CH_3$ | tert.-$C_4H_9$ | H | CH | |
| 31 | $CH_3$ | tert.-$C_4H_9$ | $CH=CH_2$ | CH | |
| 32 | $n-C_4H_9$ | tert.-$C_4H_9$ | $CH_2-C_6H_5$ | CH | |
| 33 | $n-C_4H_9$ | tert.-$C_4H_9$ | $CH_3$ | CH | |
| 34 | $n-C_4H_9$ | tert.-$C_4H_9$ | $CH=CH_2$ | CH | |
| 35 | $CH_3$ | $2,4-Cl_2-C_6H_3$ | $CH_3$ | CH | Fp. 155-156°C |
| 36 | $C_2H_5$ | $2,4-Cl_2-C_6H_3$ | $CH_3$ | CH | Fp. 144-146°C |
| 37 | $n-C_3H_7$ | $2,4-Cl_2-C_6H_3$ | $CH_3$ | CH | Öl |
| 38 | $CH_3$ | $4-Cl-C_6H_4$ | H | CH | Fp. 132-134°C |
| 39 | $C_2H_5$ | $4-Cl-C_6H_4$ | H | CH | |
| 40 | $n-C_4H_9$ | $2,4-Cl_2-C_6H_3$ | $CH_3$ | N | Fp. 98°C |
| 41 | $n-C_4H_9$ | $2,4-Cl_2-C_6H_3$ | $CH_3$ | CH | |
| 42 | $t-C_4H_9$ | $4-Cl-C_6H_4$ | $CH_3$ | N | Fp. 106-108°C |

| Nr. | $R^1$ | $R^2$ | $R^3$ | X | Physikal. Konst. |
|---|---|---|---|---|---|
| 43 | $t-C_4H_9$ | $4-Cl-C_6H_4$ | $CH_3$ | CH | |
| 44 | $t-C_4H_9$ | $2,4-Cl_2-C_6H_3$ | $CH_3$ | N | |
| 45 | $n-C_4H_9$ | $2,4-Cl_2-C_6H_4$ | H | N | Öl |
| 46 | $t-C_4H_9$ | $2,4-Cl_2-C_6CH_2$ | $CH_3$ | N | Fp. 152-154°C |
| 47 | $t-C_4H_9$ | $4-Cl-C_6H_4CH_2$ | $CH_3$ | N | Fp. 148-149°C |
| 48 | $t-C_4H_9$ | $C_6H_5CH_2$ | $CH_3$ | N | Fp. 104-105°C |
| 49 | $t-C_4H_9$ | $4-Br-C_6H_4$ | $CH_3$ | N | Fp. 133-134°C |
| 50 | $t-C_4H_9$ | $3,4-Cl_2-C_6H_3$ | $CH_3$ | N | |

Die erfindungsgemäßen Verbindungen und ihre Salze und Metallkomplexverbindungen zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden. Ferner können sie auch im Materialschutz verwendet werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Baumwolle, Soja, Kaffee, Zuckerrohr, Obst und Zierpflanzen im Gartenbau, sowie Gemüse - wie Gurken, Bohnen und Kürbisgewächse -.

Die neuen Verbindungen sind insbesondere geeignet zur Bekämpfung folgender Pflanzenkrankheiten:
Erysiphe graminis (echter Mehltau) in Getreide,

Erysiphe cichoracearum (echter Mehltau) an Kürbisgewächsen,
Podosphaera leucotricha an Äpfeln,
Uncinula necator an Reben,
Erysiphe polygoni an Bohnen,
Sphaerotheca pannosa an Rosen,
Puccinia-Arten an Getreide,
Rhizoctonia solani an Baumwolle sowie
Helminthosporiumarten an Getreide,
Ustilago-Arten an Getreide und Zuckerrohr,
Rhynchosporium secale an Getreide,
Venturia inaequalis (Apfelschorf),
Botrytis cinerea an Reben und Erdbeeren,
Piricularia oryzae an Reis.

Die Verbindungen werden angewendet, Indem man die Pflanzen mit den Wirkstoffen besprüht oder bestäubt oder die Samen der Pflanzen mit den Wirkstoffen behandelt. Die Anwendung erfolgt nach der Infektion der Pflanzen oder Samen durch die Pilze.

Die erfindungsgemäßen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwekken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Amine (z.B. Ethanolamin, Dimethylformamid) und Wasser; Trägerstoffe wie natürliche Gesteinsmehle, z.B. Kaoline, Tonerden, Talkum, Kreide und synthetische Gesteinsmehle (z.B. hochdisperse Kieselsäure, Silikate); Emulgiermittel, wie nichtionogene und anionische Emulgatoren (z.B. Polyoxyethylen - Fettalkohol - Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.-% Wirkstoff.

Die Aufwandmengen liegen je nach Art des gewünschten Effektes zwischen 0,1 und 3 kg Wirkstoff oder mehr je ha. Die neuen Verbindungen können auch im Materialschutz u.a. zur Bekämpfung holzzerstörender Pilze, wie Coniophora puteana und Polystictus versicolor eingesetzt werden. Bei der Anwendung der Wirkstoffe im Materialschutz, z.B. als Fungizide für Anstrichfarben und Weich-Polyvinylchlorid, betragen die Aufwandmengen 0,05 bis 5 Gew.-% Wirkstoff, bezogen auf das Gesamtgewicht der zu konservierenden Farben bzw. des mikrozidauszurüstenden Polyvinylchlorids. Die neuen Wirkstoffe können auch als fungizid wirksame Bestandteile öliger Holzschutzmittel zum Schutz von Holz gegen holzverfärbende Pilze eingesetzt werden. Die Anwendung erfolgt in der Weise, daß man das Holz mit diesen Mitteln behandelt, beispielsweise tränkt oder anstreicht.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:

I. Man vermischt 90 Gewichtsteile der Verbindung des Beispiels 4 mit 10 Gewichtsteilen N-Methyl-alpha-pyrrolidon und erhält eine Lösung, die zur Anwendung in Form kleinster Tropfen geeignet ist.

II. 20 Gewichtsteile der Verbindung des Beispiels 2 werden in einer Mischung gelöst, die aus 80 Gewichtsteilen Xylol, 10 Gewichtsteilen des Anlagerungsproduktes von 8 bis 10 Mol Ethylenoxid an 1 Mol ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.-% des Wirkstoffs enthält.

III. 20 Gewichtsteile der Verbindung des Beispiels 12 werden in einer Mischung gelöst, die aus 40 Gewichtsteilen Cyclohexanon, 30 Gewichtsteilen Isobutanol, 20 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.

IV. 20 Gewichtsteile der Verbindung des Beispiels 5 werden in einer Mischung gelöst, die aus 25

Gewichtsteilen Cyclohexanol, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.

V. 20 Gewichtsteile der Verbindung des Beispiels 11 werden mit 3 Gewichtsteilen des Natriumsalzes der Diisobutyl-naphthalin-alpha-sulfonsäure, 17 Gewichtsteilen des Natriumsalzes einer Ligninsulfonsäure aus einer Sulfitablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.-% des Wirkstoffs enthält.

VI. 3 Gewichtsteile der Verbindung des Beispiels 22 werden mit 97 Gewichtsteilen feinteiligem Kaolin innig vermischt. Man erhält auf diese Weise ein Stäubemittel, das 3 Gew.-% des Wirkstoffs enhält.

VII. 30 Gewichtsteile der Verbindung des Beispiels 40 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII .40 Gewichtsteile der Verbindung des Beispiels 45 werden mit 10 Teilen Natriumsalz eines Phenolsulfonsäure-harnstoff-formaldehyd-Kondensates, 2 Teilen Kieselgel und 48 Teilen Wasser innig vermischt. Man erhält eine stabile wäßrige Dispersion. Durch Verdünnen mit 100 000 Gewichtsteilen Wasser erhält man eine wäßrige Dispersion, die 0,0 Gew.-% Wirkstoff enthält.

IX. 20 Teile der Verbindung des Beispiels 4 werden mit 2 Teilen Calciumsalz der Dodecylbenzolsulfonsäure, 8 Teilen Fettalkohol-polyglykolether, 2 Teilen Natrium-salz eines Phenolsulfonsäure-harnstoff-formaldehyd-Kondensats und 68 Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise: Schwefel,

Dithiocarbamate und deren Derivate, wie Ferridimethyldithiocarbamat, Zinkdimethyldithiocarbamat, Manganethylenbisdithiocarbamat, Mangan-Zink-ethylendiamin-bis-dithiocarbamat oder Zinkethylenbisdithiocarbamat,

Tetramethylthiuramidsulfid,

Ammoniak-Komplex von Zink-(N,N-ethylen-bis-dithiocarbamat) und N,N'-Polyethylen-bis-(thiocarbamoyl)-disulfid,

Zink-(N,N'-propylen-bis-dithiocarbamat),

Ammoniak-Komplex von Zink-(N,N'-propylen-bis-dithiocarbamat) und N,N'-Propylen-bis(thiocarbamoyl)-disulfid;

Nitroderivate, wie

Dinitro-(1-methylheptyl)-phenylcrotonat,

2-sec-Butyl-4,6-dinitrophenyl-3,3-dimethylacrylat,

2-sec-Butyl-4,6-dinitrophenyl-isopropylcarbonat;

heterocyclische Substanzen, wie N-Trichlormethylthio-tetrahydrophthalimid,

N-(1,1,2,2-Tetrachlorethylthio)-tetrahydrophthalimid,

N-Trichlormethylthio-phthalimid,

2-Heptadecyl-2-imidazolin-acetat,

2,4-Dichlor-6-(o-chloranilino)-s-triazin,

0,0-Diethyl-phthalimidophosphonothioat,

5-Amino-1-(bis-(dimethylamino)-phosphinyl)-3-phenyl-1,2,4--triazol,

5-Ethoxy-3-trichlormethyl-1,2,4-thiadiazol,

2,3-Dicyano-1,4-dithioanthrachinon,

2-Thio-1,3-dithio-(4,5-b)-chinoxalin,

1-(Butylcarbamoyl)-2-benzimidazol-carbaminsäuremethylester,

2-Methoxycarbonylamino-benzimidazol,

2-Rhodanmethylthio-benzthiazol,

4-(2-Chlorphenylhydrazono)-3-methyl-5-isoxazolan,

Pyridin---thio-1-oxid,
8-Hydroxychinolin bzw. dessen Kupfersalz,
2,3-Dihydro-5-carboxanilido-6-methyl-1,4-oxathiin-4,4--dioxid,
2,3-Dihydro-5-carboxanilido-6-methyl-1,4-oxathiin,
2-(Furyl-(2))-benzimidazol,
Piperazin-1,4-diyl-bis-(1-(2,2,2-trichlor-ethyl)-formamid),
2-(Thiazolyl-(4))-benzimidazol,
5-Butyl-2-dimethylamino-4-hydroxy-6-methyl-pyrimidin,
Bis-(p-Chlorphenyl)-pyridinmethanol,
1,2-Bis-(3-ethoxycarbonyl-2-thioureido)-benzol,
1,2-Bis-(3methoxycarbonyl-2-thioureido)-benzol und weitere Substanzen, wie Dodecylguanidinacetat,
3-(3-(3,5-Dimethyl-2-oxycyclohexyl)-2-hydroxyethyl)--glutaramid,
Hexachlorbenzol,
N-Dichlorfluormethylthio-N',N'-dimethyl-N-phenyl-schwefel-säurediamid,
2,5-Dimethyl-furan-3-carbonsäureanilid,
2,5-Dimethyl-furan-3-carbonsäure-cyclohexylamid,
2-Cyan-N-(ethylaminocarbonyl)-2-(methoxyimino)-acetamid,
2-Methyl-benzoesäure-anilid,
2-Iod-benzoesäure-anilid,
1-(3,4-Dichloranilino)-1-formylamino-2,2,2-tricploretpan,
2,6-Dimethyl-N-tridecyl-morpholin bzw. dessen Salze, 2,6-Dimethyl-N-cyclododecyl-morpholin bzw. dessen Salze,
DL-Methyl-N-(2,6-dimethyl-phenyl)-N-furoyl(2)-alaninat,
DL-N-(2,6-Dimethyl-phenyl)-N-(2'-methoxyacetyl)-alanin--methylester,
5-Nitro-isophthalsäure-di-isopropylester,
1-(1',2',4'-Triazolyl-1')-(4'chlorphenoxy)-3,3-dimethyl-butan-2-on,
1-(1',2' ,4'-Triazolyl-1')-1-(4'-chlorphenoxy)-3,3-dimethyl-butan-2-ol,
N-(2,6-Dimethylphenyl)-N-chloracetyl-D,L-2-aminobutyro-lacton,
N-(n-Propyl)-N-(2,4,6-trichlorphenoxyethyl)-N'-imidazolyl-harnstoff.
DL-N-(2,6-Dimethylphenyl)-N-(phenylacetyl)-alaninmethylester
5-Methyl-5-vinyl-3-(3,5-dichlorphenyl)-2,4-dioxo-1,3-oxa-zolidin
3-(3,5-Dichlorphenyl)-5-methyl-5-methoxymethyl-1,3-oxa-zolidin-2,4-dion
3-(3,5-Dichlorphenyl)-1-isopropyl-carbamoylhydantoin N-(3,5-Dichlorphenyl)-1,2-dimethyl-cyclopropan-1,2-di-carbonsäureimid
N-[3-(p-tert.-Butylphenyl)-2-methylpropyl]-cis-2,6-di-methylmorpholin
1-[2-(2,4-Dichlorphenyl)-4-ethyl-1,3-dioxolan-2-yl-ethyl]--1H-1,2,4-triazol
2-[2-(2,4-Dichlorphenyl)-4-n-propyl-1,3-dioxolan-2-yl--ethyl]-1H-1,2,4-triazol

Die folgenden Beispiele zeigen die biologische Wirkung der neuen Substanzen. Als Vergleichssubstanz diente das aus der DE-OS 26 40 823 bekannte O-Ethyl-N-(1,2,4-Triazol-1--yl)-p-chlorbenzaldehydacetal (Z).

Beispiel B

Wirksamkeit gegen Weizenbraunrost

Blätter von in Töpfen gewachsenen Weizensämlingen der Sorte "Jubilar" werden mit Sporen des Braunrostes (Puccinia recondita) bestäubt. Danach werden die Töpfe für 24 h bei 20 bis 22 °C in eine Kammer mit hoher Luftfeuchtigkeit (90 bis 95 %) gestellt. Während dieser Zeit keimen die Sporen aus und die Keimschläuche dringen in das Blattgewebe ein. Die infizierten Pflanzen werden anschließend mit wäßrigen Spritzbrühen, die 80 % Wirkstoff und 20 % Emulgiermittel (Polyoxyethylensorbitanmonooleat) in der Trockensubstanz enthalten, tropfnaß gespritzt. Nach dem Antrocknen des Spritzbelages werden die Versuchspflanzen im Gewächshaus bei Temperaturen zwischen 20 und 22 °C und 65 bis 70 % relativer Luftfeuchte aufgestellt. Nach 8 Tagen wird das Ausmaß der Rostpilzentwicklung auf den Blättern ermittelt.

In diesem Versuch zeigten beispielsweise die Substanzen der Beispiele 9, 11, 12 und 40 bei der Anwendung als 0,025 %ige Spritzbrühe eine bessere fungizide Wirkung (z.B. 90 %) als Z (z.B. 50 %).

Beispiel C

Wirksamkeit gegen Gurkenmehltau

Blätter von in Töpfen gewachsenen Gurkenkeimlingen der Sorte "Chinesische Schlange" werden im Zweibettstadium mit einer Suspension von Sporen des Gurkenmehltaus (Erysiphe cichoracearum besprüht. Nach etwa 20 h werden die Versuchspflanzen mit wäßriger Spritzbrühe, die 80 % Wirkstoff und 20 % Emulgiermittel (Polyoxyethylensorbitanmonooleat) in der Trockensubstanz enthält, bis zur Tropfnässe besprüht. Nach dem Antrocknen des Spritzbelages werden sie anschließend im Gewächshaus bei Temperaturen zwischen 20 und 22°C und 70 bis 80 % relativer Luftfeuchtigkeit aufgewird das Ausmaß der Pilzentwicklung nach 21 Tagen beurteilt.

In diesem Versuch zeigten beispielsweise die Substanzen der Beispiele 5, 11, 12, 22, 35, 36, 40, 42 und 45 bei Anwendung als 0,025 %ige Spritzbrühe eine gute fungizide Wirkung (z.B. 100 %).

## Ansprüche

1. Verfahren zur direkten Bekämpfung von Pilzen, **dadurch gekennzeichnet**, daß man eine fungizid wirksame Menge eines alpha-Azolylglykols der Formel I

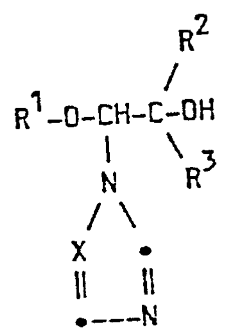

(I),

in welcher

$R^1$ $C_1$-$C_6$-Alkyl,

$R^2$ $C_1$-$C_6$-Alkyl oder gegebenenfalls durch Halogen substituiertes Phenyl oder Biphenyl,

$R^3$ Wasserstoff, $C_1$-$C_6$-Alkyl, $C_2$-$C_6$-Alkenyl, $C_2$-$C_6$-Alkinyl oder gegebenenfalls durch Halogen substituiertes Benzyl und

X CH oder N bedeutet

oder dessen für Pflanzen verträgliches Salz oder Metallkomplex auf Pilze einwirken läßt.

2. Verfahren nach Anspruch 1, **dadurch gekennzeichnet**, daß man eine Verbindung der Formel I gemäß Anspruch 1 verwendet, in der $R^1$ $C_1$-$C_6$-Alkyl, $R^2$ Halogenphenyl und $R^3$ Wasserstoff oder Methyl bedeuten.

3. Verwendung eines alpha-Azolylglykols der Formel I

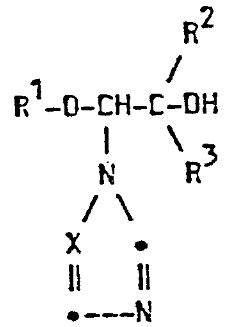

(I),

in welcher

$R^1$ $C_1$-$C_6$-Alkyl,

$R^2$ $C_1$-$C_6$-Alkyl oder gegebenenfalls durch Halogen substituiertes Phenyl oder Biphenyl,

$R^3$ Wasserstoff, $C_1$-$C_6$-Alkyl, $C_2$-$C_6$-Alkenyl, $C_2$-$C_6$-Alkinyl oder gegebenenfalls durch Halogen substituiertes Benzyl und

X CH oder N bedeutet,

oder dessen für Pflanzen verträglichen Salzes oder Metallkomplexes zur direkten Bekämpfung von Pilzen.

**4.** Verwendung nach Anspruch 3, **dadurch gekennzeichnet**, daß man eine Verbindung der Formel I gemäß Anspruch 1 verwendet, in der $R^1$ $C_1$-$C_6$-Alkyl, $R^2$ Halogenphenyl und $R^3$ Wasserstoff oder Methyl bedeuten.

## Claims

**1.** A method for directly controlling fungi, wherein a fungicidal amount of an alpha-azolylglycol of the formula I

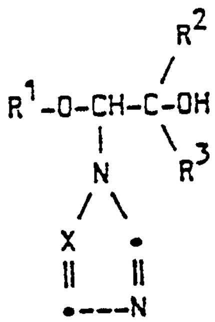

(I)

where $R^1$ is $C_1$-$C_6$-alkyl, $R^2$ is $C_1$-$C_6$-alkyl or unsubstituted or halogen-substituted phenyl or biphenyl, $R^3$ is hydrogen, $C_1$-$C_8$-alkyl, $C_2$-$C_8$-alkenyl, $C_2$-$C_6$-alkynyl or unsubstituted or halogen-substituted benzyl and X is CH or N, or of its plant-tolerated salt or metal complex is allowed to act on fungi.

**2.** A method as claimed in claim 1, wherein a compound of the formula I as claimed in claim 1 is used, in which $R^1$ is $C_1$-$C_6$-alkyl, $R^2$ is halophenyl and $R^3$ is hydrogen or methyl.

**3.** Use of an alpha-azolylglycol of the formula I

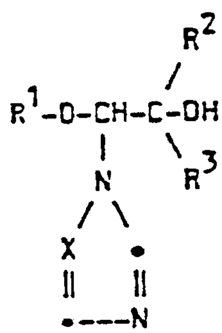

(I)

where $R^1$ is $C_1$-$C_6$-alkyl, $R^2$ is $C_1$ -$C_6$-alkyl or unsubstituted or halogen-substituted phenyl or biphenyl, $R^3$ is hydrogen, $C_1$-$C_6$-alkyl, $C_2$-$C_6$-alkenyl, $C_2$-$C_6$-alkynyl or unsubstituted or halogen-substituted benzyl and X is CH or N, or of its plant-tolerated salt or metal complex for directly controlling fungi.

**4.** Use as claimed in claim 3, wherein a compound of the formula I as claimed in claim I is used, in which $R^1$ is $C_1$-$C_6$-alkyl, $R^2$ is halophenyl and $R^3$ is hydrogen or methyl.

## Revendications

**1.** Procédé de lutte directe contre les champignons, caractérisé par le fait que l'on fait agir sur les

champignons une quantité efficace du point de vue fongicide d'un alpha-azolylglycol de formule I

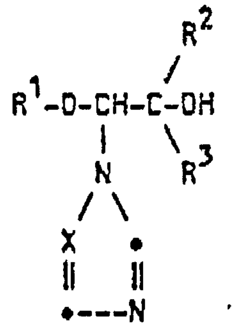

(I)

dans laquelle

$R^1$ représente alkyle en $c_1$-$c_6$,

$R^2$ , alkyle en $c_1$-$c_6$ ou phényle Ou biphényle éventuellement substitués par halogène,

$R^3$, hydrogène, alkyle en $C_1$-$C_6$, alcényle en $c_2$-$c_6$, alcynyle en $C_2$-$C_6$ ou benzyle èventuellement substitué par halogène,

X, CH ou N,

ou de son sel ou complexe métallique compatibles avec les plantes.

**2.** Procédé selon la revendication 1, caractérisé par le fait que l'on utilise un composé de formule I, selon la revendication 1, dans laquelle $R^1$ représente alkyle en $C_1$-$C_6$, $R^2$, halogénophényle et $R^3$, hydrogène ou méthyle.

**3.** Utilisation d'un alpha-azolylglycol de formule I

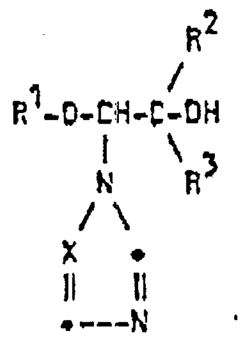

(I)

dans laquelle

$R^1$ représente alkyle en $C_1$-$C_6$,

$R^2$, alkyle en $C_1$-$C_6$ ou Phényle ou biphényle éventuellement substitués par halogène,

$R^3$, hydrogène, alkyle en $C_1$-$C_6$ alcényle en $C_2$-$C_6$, alcynyle en $C_2$-$C_6$ ou benzyle éventuellement substitué par halogène,

X, CH ou N,

ou de son sel ou complexe métallique tolérés par les plantes, pour la lutte directe contre les champignons.

**4.** Utilisation selon la revendication 3, caractérisée par le fait que l'on utilise un composé de formule I, selon la revendication 1, dans laquelle $R^1$ représente alkyle en $C_1$-$C_6$, $R^2$, halogénophényle et $R^3$, hydrogène ou méthyle.